(19)

**Europäisches Patentamt**
**European Patent Office**
**Office européen des brevets**

(11) **EP 4 756 719 A1**

(12) **EUROPEAN PATENT APPLICATION**

(43) Date of publication:
**10.06.2026 Bulletin 2026/24**

(21) Application number: **24217642.8**

(22) Date of filing: **05.12.2024**

(51) International Patent Classification (IPC):
**G06T 11/00** $^{(2026.01)}$ **G06T 7/20** $^{(2017.01)}$
**A61B 6/03** $^{(2006.01)}$

(52) Cooperative Patent Classification (CPC):
**G06T 12/00; A61B 6/032; A61B 6/5264;**
**G06T 7/20;** G06T 2207/10081; G06T 2211/412

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicant: **Koninklijke Philips N.V.**
**5656 AE Eindhoven (NL)**

(72) Inventors:
• **KOEHLER, Thomas**
**Eindhoven (NL)**

• **GRASS, Michael**
**Eindhoven (NL)**
• **SCHUELKE, Christophe Michael Jean**
**5656AG Eindhoven (NL)**
• **KABUS, Sven**
**Eindhoven (NL)**
• **WILD, Sebastian**
**Eindhoven (NL)**

(74) Representative: **Philips Intellectual Property &**
**Standards**
**High Tech Campus 52**
**5656 AG Eindhoven (NL)**

(54) **CT MOTION COMPENSATION**

(57) Proposed concepts thus aim to provide schemes, solutions, concept, designs, methods and systems pertaining to compensating for motion during a CT scan. In particular, embodiments aim to provide a method for compensating for motion during a CT scan by obtaining two CT scan volumes of the same portion of a subject (and which at least partially overlap in time) and then taking one slice of one of the CT scan volumes and generating a reference motion state (i.e., a ground truth reference) based on the subject's motion state at the point of time of the selected slice. Based on this reference motion state and the first and second CT scan volumes, a time-dependent rigid transformation function can be generated (i.e., a transformation function which only uses rigid transformations) and then either applied directly to the first and second CT scan volumes to compensate for their motion or applied to the original projection data to generate a motion-compensated CT scan volume from scratch.

**FIG. 1**

EP 4 756 719 A1

**Description**

FIELD OF THE INVENTION

This invention relates to the field of motion compensation, and more specifically, to the field of motion compensation for computed tomography.

BACKGROUND OF THE INVENTION

Standard CT reconstruction algorithms typically assume the object/subject to be static. These algorithms achieve, to some extent, robustness with respect to motion via the smooth weighting of redundant data. However, this approach mainly reduces the extent of motion artifacts, and smoothing still happens if the subject moves during data acquisition.

The simplest method to handle motion during a scan is the retrospective gating approach, which can be used if the motion is periodic. This is mainly used in cardiac CT and pulmonary-gated CT. Still, even with gating, residual motion can occur within the gating window.

Recently, however, more advanced methods for handling subject motion have been formulated, such as motion compensated reconstruction (MCR). This has been established for cardiac CT and pulmonary-gated CT.

For non-periodic pulmonary motion, it is also known to reconstruct several images from the raw data with slight temporal displacement by using data from different detector areas (e.g., only the lower/upper half of it) in different reconstructions. An elastic registration can then subsequently be applied to estimate the relative motion between the reconstructions, followed by a conventional MCR back-projection. Due to the technique used to generate the input images for registration (i.e., only using data from part of the detector), this method is referred to as detector segment based MCR.

SUMMARY OF THE INVENTION

The invention is defined by the claims.

According to examples in accordance with an aspect of the invention, there is provided a method for compensating for motion during a CT scan.

The method comprises: receiving a first CT scan volume of a portion of a subject comprising a first stack of slices separated in time; receiving a second CT scan volume of the same portion of a subject comprising a second stack of slices separated in time, wherein the second CT scan volume partially overlaps in time with the first CT scan volume; generating a reference motion state based on one of the slices from the first stack of slices; determining a time-dependent rigid transformation function based on the reference motion state and the first and second CT scan volumes; and generating a motion-compensated CT scan volume based on the rigid transformation function and either the first and second CT scan volumes or projection data from which the first and second CT scan volumes were reconstructed.

Proposed concepts thus aim to provide schemes, solutions, concepts, designs, methods, and systems pertaining to compensating for motion during a CT scan. In particular, embodiments aim to provide a method for compensating for motion during a CT scan by obtaining two CT scan volumes of the same portion of a subject (and which at least partially overlap in time) and then taking one slice of one of the CT scan volumes and generating a reference motion state (i.e., a ground truth reference) based on the subject's motion state at the point of time of the selected slice. Based on this reference motion state and the first and second CT scan volumes, a time-dependent rigid transformation function can be generated (i.e., a transformation function which only uses rigid transformations) and then either applied directly to the first and second CT scan volumes to compensate for their motion or applied to the original projection data to generate an entirely new motion-compensated CT scan volume.

In other words, it is proposed that by generating a particular reference motion state from the two received CT scan volumes (e.g., by merely selecting a single slice from one of the CT scan volumes), a rigid transformation function can be determined which can essentially be used to rigidly transform every other slice in the stacks of slices of the two CT scan volumes to match the reference motion state. After generating this rigid transformation function, the two scan volumes can then be 'corrected' with said function, such that all the slices will then match the reference motion state. Alternatively, the rigid transformation function can be used to compensate for motion during a fresh reconstruction from the original projection data (from which the CT scan volumes were reconstructed).

By specifically using a rigid transformation function, that is a transformation function which only uses rigid transformations, i.e., translations and/or rotations without any scaling or deformation, motion of rigid body parts (like the head) can be more efficiently compensated. In other words, this method of motion compensation assumes that motion of the portion of the subject being imaged is rigid, and this assumption allows for an easier, effective compensation of motion. This may be particularly useful, for example, for CT scans of rigid parts of the body such as the head. For example, head motion can be effectively assumed to be rigid motion, moving as a single, solid entity without any deformation or change in shape.

Ultimately, an improved method for compensating for motion during a CT scan is provided which allows for motion to be compensated for by assuming the motion to be rigid.

In some embodiments, determining the rigid transformation function may comprise generating a first estimate of the rigid transformation function based on the reference motion state and a first neighboring slice in the second stack of slices neighboring the slice upon which the reference motion state was based. A neighboring slice can be understood as a slice which corresponds in position rather than in time, and as the two stacks of slices are time offset from one another (i.e., only partially overlapping in time), this thus leads to a time difference between the neighboring slices, such that an estimate of the (time-dependent) rigid transformation function may be generated based on the differences between the two neighboring slices. Starting with the slice upon which the reference motion state was based may facilitate the estimation of a rigid transformation function which can compensate for motion which deviates from the reference motion state.

In some embodiments, determining the rigid transformation function may further comprise generating a second estimate based on the first estimate, a first corresponding slice in the first stack of slices corresponding in time to the first neighboring slice, and a second neighboring slice in the second stack of slices neighboring the first corresponding slice. The first estimate of the rigid transformation function may thus be built on, using a slice from the first stack corresponding in time to the first neighboring slice (i.e., in the same motion state), and then a neighboring slice again. As would be apparent to the skilled person, this process of iterative estimation at different points in time can be chained together, wherein the next estimate is based on the previous estimate, a slice corresponding in time to the previous neighboring slice, and then a new neighboring slice.

In some embodiments, determining the rigid transformation function may further comprise interpolating between the first estimate and the second estimate. This may facilitate the inference of the rigid transformation function for time-states in between those for which the rigid transformation function has been directly estimated.

In some embodiments, generating a motion-compensated CT scan volume may comprise: compensating for motion in each slice of the first and second stacks of slices respectively based on the rigid transformation function to generate motion-compensated first and second CT scan volumes; and generating a motion-compensated CT scan volume based on the motion-compensated first and second CT scan volumes. This may facilitate the motion in the first and second CT scan volumes being directly compensated for in order to generate the motion-compensated CT scan volume.

In some embodiments, generating a motion-compensated CT scan volume may comprise: receiving projection data from which the first and second CT scan volumes were reconstructed; and reconstructing a motion-compensated CT scan volume based on the projection data and the rigid transformation function. This may allow a motion-compensated CT scan volume to be freshly reconstructed from the original projection data, taking advantage of the determined rigid transformation function during the reconstruction process.

In some embodiments, the first stack of slices may comprise at least one slice which is earlier than any slice in the second stack of slices, and the reference motion state may be generated based on one of these earlier slices. This may allow the reference motion state to be based on one of the earliest slices (if not the earliest slice), where the subject is likely to be in a more preferable position, e.g., before any unwanted movement.

In some embodiments, determining the rigid transformation function may comprise estimating the second CT scan volume to be separated in time from the first CT scan volume by a constant corresponding to an integer number of slices. This may facilitate more efficient and/or effective determining of neighboring slices, for example, as well as interpolation.

In some embodiments, generating the reference motion state may comprise selecting one of the slices from the first stack of slices as the reference motion state. Simply selecting one of the slices is an efficient and sufficient way of generating the reference motion state.

In some embodiments, generating the reference motion state may further comprise transforming the selected slice. This may allow for the selected slice to be translated or rotated, for example, allowing for more flexibility in generating the reference motion state.

In some embodiments, a rigid transformation may comprise a translation and/or a rotation without any scaling or deformation.

In some embodiments, the portion of the subject may comprise a rigid portion of the subject. The present method may be of particular benefit in compensating for motion of a rigid portion of a subject.

In some embodiments, the rigid portion of the subject may be the head of the subject. The present method may be of particular benefit in compensating for motion of a subject's head.

According to another aspect of the invention, there is provided a computer program product comprising computer program code means which, when executed on a computing device having a processing system, cause the processing system to perform all of the steps of the computer-implemented method according to any herein disclosed method.

According to another aspect of the invention, there is provided a system for compensating for motion during a CT scan. The system comprising an input interface configured to: receive a first CT scan volume of a portion of a subject comprising a first stack of slices separated in time; and receive a second CT scan volume of the same portion of a subject comprising a second stack of slices separated in time, wherein the second CT scan volume partially overlaps in time with the first CT scan volume; and a processing unit configured to: generate a reference motion state based on one of the slices from the first stack of slices; determine a time-dependent rigid transformation function based on the reference motion state and the first and second CT scan volumes; and generate a motion-compensated CT scan volume based on the rigid transformation function and either the first and second CT scan volumes or projection data from which the first and second CT scan volumes were reconstructed.

In some embodiments, determining the rigid transformation function may comprise generating a first estimate of the rigid transformation function based on the reference motion state and a first neighboring slice in the second stack of slices neighboring the slice upon which the reference motion state was based.

Thus, there may be proposed concepts for compensating for motion during a CT scan, and this may be done based on two CT scan volumes partially overlapping in time and the determining of a time-dependent rigid transformation function.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 is a simplified flow diagram of a method for compensating for motion during a CT scan according to a proposed embodiment;
Fig. 2A is an illustration showing an example of how a rigid transformation function can be used to compensate for motion in each slice of three CT scan volumes, according to a proposed embodiment;
Fig. 2B is an illustration showing an example of how a first and second estimate of a rigid transformation function can be generated, according to a proposed embodiment;
Fig. 3 is a flow diagram of a method for compensating for motion during a CT scan according to a proposed embodiment;
Fig. 4 is a flow diagram of a method for compensating for motion during a CT scan according to a proposed embodiment;
Fig. 5 is a simplified block diagram of a system for compensating for motion during a CT scan according to a proposed embodiment; and
Fig. 6 illustrates an example of a computer within which one or more parts of an embodiment may be employed.

DETAILED DESCRIPTION OF THE EMBODIMENTS

The invention will be described with reference to the Figures.

Embodiments of the invention aim to provide a method for compensating for motion during a CT scan. This can be achieved by obtaining two CT scan volumes of the same portion of a subject (and which at least partially overlap in time) and then taking one slice of one of the CT scan volumes and generating a reference motion state (i.e., a ground truth reference) based on the subject's motion state at the point of time of the selected slice. Based on this reference motion state and the first and second CT scan volumes, a time-dependent rigid transformation function can be generated (i.e., a transformation function which only uses rigid transformations) and then either applied directly to the first and second CT scan volumes to compensate for their motion or applied to the original projection data to generate an entirely new motion-compensated CT scan volume.

In other words, it is proposed that by generating a particular reference motion state from the two received CT scan volumes (e.g., by merely selecting a single slice from one of the CT scan volumes), a rigid transformation function can be determined which can essentially be used to rigidly transform every other slice in the stacks of slices of the two CT scan volumes to match the reference motion state. After generating this rigid transformation function, the two scan volumes can then be 'corrected' with said function, such that all the slices will then match the reference motion state. Alternatively, the rigid transformation function can be used to compensate for motion during a fresh reconstruction from the original projection data (from which the CT scan volumes were reconstructed).

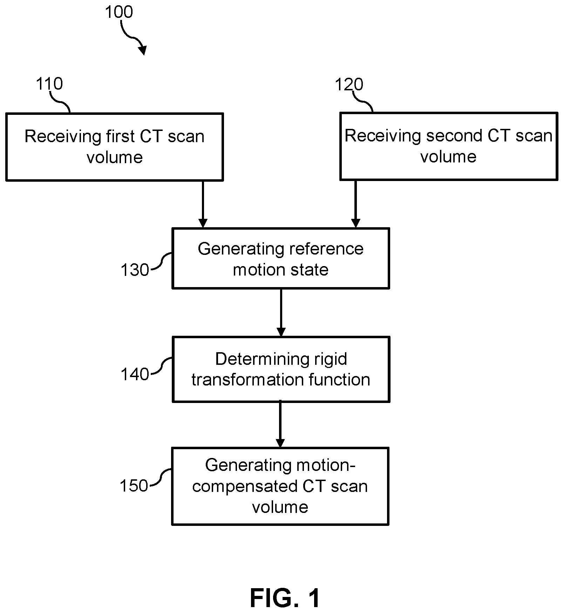

Referring now to Fig. 1, there is depicted a simplified flow diagram of a method 100 for compensating for motion during a CT scan according to a proposed embodiment.

Step 110 comprises receiving a first CT scan volume of a portion of a subject comprising a first stack of slices separated in time and step 120 comprises receiving a second CT scan volume of the same portion of a subject comprising a second stack of slices separated in time, wherein the second CT scan volume partially overlaps in time with the first CT scan volume. For example, each CT scan volume is an overlapping sub-portion of the same projection data. The stacks of slices being separated in time can be understood as, within each stack, each slice having a different time stamp, e.g., separated by time constant.

In this embodiment, the portion of the subject comprises a rigid portion of the subject. The present method may be of particular benefit in compensating for motion of a rigid portion of a subject. The skilled person would appreciate, however, that the present invention would also be of use when the portion of the subject comprises a not-strictly rigid portion of the subject, for instance, a semi- or partially rigid portion of the subject.

In fact, in this embodiment, the rigid portion of the subject is specifically the head of the subject. The present invention is of particular benefit in compensating for motion of a subject's head, as the head is one of the most rigid portions of the body.

Step 130 comprises generating a reference motion state based on one of the slices from the first stack of slices. This reference motion state can be understood as the motion state that will form the ground truth of the motion compensation. In other words, motion will be compensated for in slices in order to have their motion state match the reference motion state. As the reference motion state is based on one of the slices, the time stamp of this selected slice can then be thought of as a reference time stamp.

It is noted, in this embodiment, that the first stack of slices comprises at least one slice which is earlier than any slice in the second stack of slices, and the reference motion state is generated based on one of these earlier slices. This can thus allow the reference motion state to be based on one of the earliest slices (if not the earliest slice), where the subject is likely to be in a more preferable position, e.g., before any unwanted movement. As would be understood by the skilled person, however, this feature is not essential and in other embodiments, the reference motion state can be based on any suitable slice, regardless of its relative timing.

Step 140 comprises determining a time-dependent rigid transformation function based on the reference motion state and the first and second CT scan volumes. The rigid transformation function can be understood as a function dependent on time that can be used to transform the motion states of slices to the reference motion state. In other words, if a slice has a time stamp that is not the reference time stamp, the rigid transformation function can be used to essentially calculate how to transform said slice such that its motion state matches the reference motion state. As the rigid transformation function is time-dependent, depending on the time stamp of the slice being transformed, different transformations can be applied to have the motion state of the slice then match the reference motion state.

To be clear, a rigid transformation can comprise a translation and/or a rotation without any scaling or deformation. If any scaling or deformation is performed, then it is no longer a rigid transformation.

Step 150 comprises generating a motion-compensated CT scan volume based on the rigid transformation function and either the first and second CT scan volumes or projection data from which the first and second CT scan volumes were reconstructed. In other words, the rigid transformation function can be used to generate a motion-compensated CT scan volume either by applying it directly to the first and second CT scan volumes to compensate for their motion or by applying it to the original projection data to generate a motion-compensated CT scan volume from scratch.

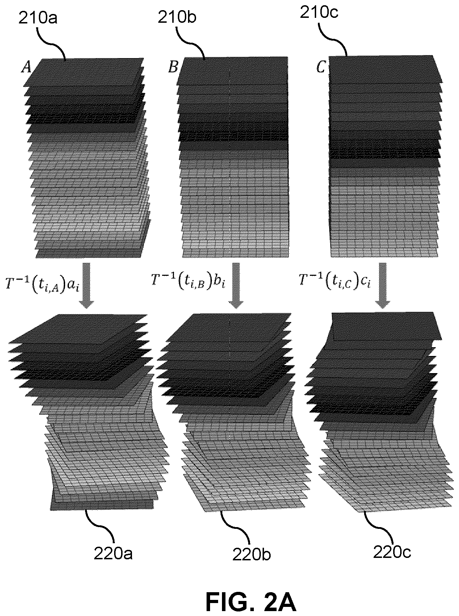

Referring now to Fig. 2A, there is depicted an illustration showing an example of how a rigid transformation function can be used to compensate for motion in each slice of three CT scan volumes, according to a proposed embodiment.

At the top, there can be seen three non-motion-corrected CT scan volumes, 210a, 210b, and 210c. For example, these can be CT scan volumes reconstructed from a helical acquisition using three different parts of the detector. These three CT scan volumes each comprise stacks of slices separated in time, respectively. Furthermore, these three CT scan volumes all partially overlap in time with one another (as indicated by the shading of the slices, wherein the same shade corresponds to the same timestamp). For instance, since the detector segments cause a certain temporal shift of the image slices, the timestamps are shifted between the three volumes. After the time-dependent rigid transformation function has been determined, it is applied to each volume independently, as indicated by the downward arrows, resulting in three motion-corrected CT scan volumes, 220a, 220b, and 220c. As can be seen, each slice in in the motion-compensated volumes has been rotated and/or translated compared to the non-motion-compensated volumes, 210a, 210b, and 210c. In other words, Fig. 2A shows the transformation of CT scan volumes 210a, 210b, and 210c to a ground truth by a slice-wise application of a rigid transformation function. While the transformations in the example shown in Fig. 2A are 2D motions for illustrative purposes, it is also envisioned that 3D motion is estimated for each slice.

As would be appreciated by the skilled person, the slices which correspond in time to one another (i.e., which share the same timestamp (and thus shade)) are transformed in the same way, as the differences between their motion states and the reference motion state will be the same.

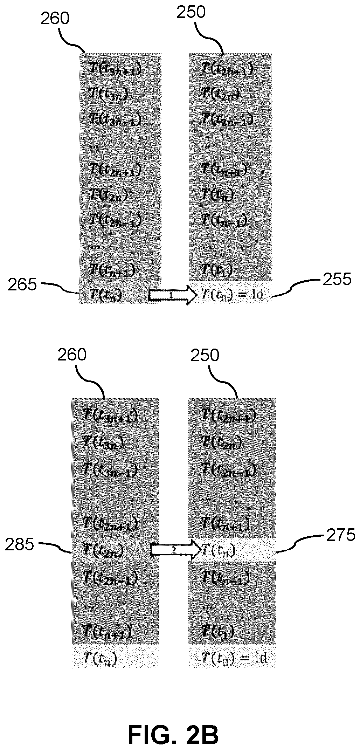

Referring now to Fig. 2B, there is depicted an illustration showing an example of how a first and second estimate of a rigid transformation function can be generated, according to a proposed embodiment.

There is provided a first CT scan volume 250 comprising a first stack of slices separated in time and a second CT scan volume 260 comprising a second stack of slices separated in time.

For instance, determining the rigid transformation function can comprise generating a first estimate of the rigid transformation function based on the reference motion state (in this case, simply selected as the first slice 255 of the first CT scan volume 250) and a first neighboring slice 265 (i.e., the first slice 265 of the second CT scan volume 260) neighboring the slice 255 upon which the reference motion state was based (again, in this case, simply selected as). A neighboring slice can be understood as a slice corresponding in position (i.e., first, second, third, etc.) rather than time. This thus results in the two slices being time offset from one another as the volumes themselves, 250 and 260, are time offset from one another.

Determining the rigid transformation function can then further comprise generating a second estimate of the rigid transformation function based on the first estimate, a first corresponding slice 275 in the first stack of slices 250, corresponding in time (i.e., having the same timestamp) as the first neighboring slice 265, and a second neighboring slice 285 in the second stack of slices 260 neighboring the first corresponding slice 275.

To be clear, the first and second estimations are thus estimations of the same time-dependent rigid transformation function at a first and second time, respectively - the first time corresponding to the timestamp of first neighboring slice 265 and the second time corresponding to the timestamp of the second neighboring slice 285.

Determining the rigid transformation function can then further comprise interpolating for the slices between the first estimate and the second estimate. This can allow the rigid transformation function for timestamps in between those for which the rigid transformation function has been directly estimated to be inferred. As the skilled person would understand, interpolation would only be required/possible if the two CT scan volumes are separated in time by more than one slice, as otherwise, there would then be no time-states for which estimates are not directly generated.

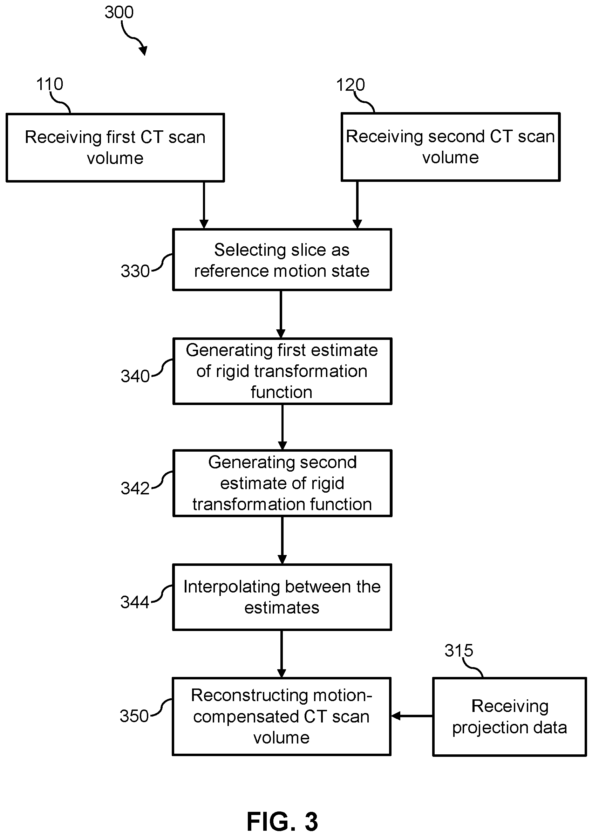

Referring now to Fig. 3, there is depicted a flow diagram of a method 300 for compensating for motion during a CT scan according to a proposed embodiment.

Steps 110 and 120 are substantially the same as have been described in relation to the method 100 in Fig. 1.

Step 330 comprises selecting one of the slices from the first stack of slices as the reference motion state. In other words, in this embodiment, generating the reference motion state comprises selecting one of the slices from the first stack of slices as the reference motion state. Simply selecting one of the slices is an efficient and sufficient way of generating the reference motion state. In other embodiments, however, the reference motion state can be generated in any suitable way, as would be understood by the skilled person, for example, after selecting a slice, the selected slice could be transformed. For example, this can allow the selected slice to be translated or rotated, for example, allowing for more flexibility in generating the reference motion state.

Step 340 comprises generating a first estimate of the rigid transformation function based on the reference motion state and a first neighboring slice in the second stack of slices neighboring the slice upon which the reference motion state was based (as described above in relation to Fig. 2B). As stated, a neighboring slice can be understood as a slice which corresponds in position rather than time, and as the two stacks of slices are time offset from one another (i.e., only partially overlapping in time), this thus leads to a time difference between the neighboring slices, such that an estimate of the (time-dependent) rigid transformation function can be generated based on the differences between the two neighboring slices. Starting with the slice upon which the reference motion state was based facilitates the estimation of a rigid transformation function which can compensate for motion which deviates from the reference motion state.

Step 342 comprises generating a second estimate based on the first estimate, a first corresponding slice in the first stack of slices corresponding in time to the first neighboring slice, and a second neighboring slice in the second stack of slices neighboring the first corresponding slice (as described above in relation to Fig. 2B). The first estimate of the rigid transformation function can thus be built on, using a slice from the first stack corresponding in time to the first neighboring slice (i.e., in the same motion state), and then a neighboring slice again. As would be apparent to the skilled person, this process of iterative estimation at different points in time can be chained together, wherein the next estimate is based on the previous estimate, a slice corresponding in time to (i.e., having the same timestamp as) the previous neighboring slice, and then a new neighboring slice (i.e., having the same position in their respective stacks).

Step 344 then comprises interpolating between the first estimate and the second estimate. This allows for the rigid transformation function to be inferred for time-states in between those for which the rigid transformation function has been directly estimated.

It is noted that in this embodiment, before steps 340, 342, and 344, it is estimated that the second CT scan volume is separated in time from the first CT scan volume by a constant corresponding to an integer number of slices. This facilitates more efficient and/or effective determining of neighboring slices, for example, as well as interpolation, though is not essential to the working of the invention.

Step 315 comprises receiving projection data from which the first and second CT scan volumes were reconstructed. Receiving can also be understood as obtaining, retrieving, etc., and is not intended to limit to any particular way of getting hold of the projection data.

Step 350 thus comprises reconstructing a motion-compensated CT scan volume based on the projection data and the rigid transformation function. This essentially allows a motion-compensated CT scan volume to be freshly reconstructed from the original projection data, taking advantage of the determined rigid transformation function during the reconstruction process.

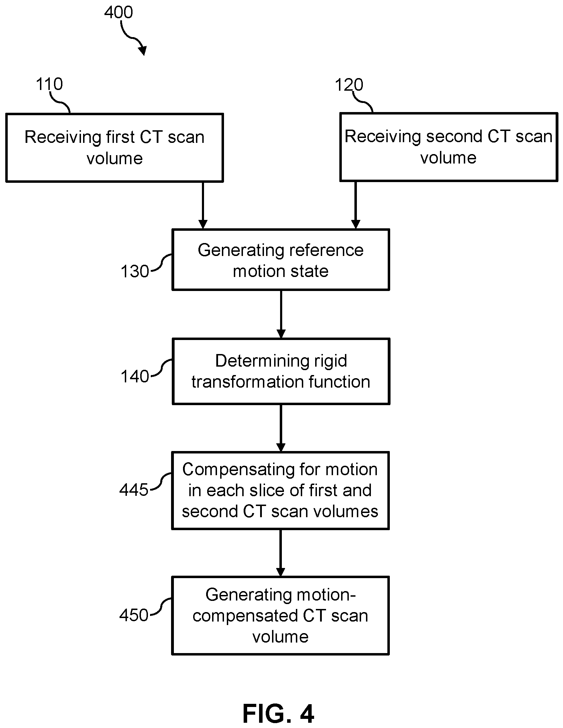

Referring now to Fig. 4, there is depicted a flow diagram of a method 400 for compensating for motion during a CT scan according to a proposed embodiment. Steps 110, 120, 130 and 140 are substantially the same has have been described in relation to method 100 of Fig. 1.

Step 445 comprises compensating for motion in each slice of the first and second stacks of slices respectively based on the rigid transformation function to generate motion-compensated first and second CT scan volumes. Step 450 then comprises generating a motion-compensated CT scan volume based on the motion-compensated first and second CT scan volumes, for example, combining them in any suitable way, as would be understood by the skilled person. This thus facilitates the motion in the first and second CT scan volumes being directly compensated for in order to generate the motion-compensated CT scan volume.

Now, considering a specific example, during the CT scan, the object-being-scanned undergoes a rigid motion, i.e., the object is transformed by a time-dependent translation and rotation. This transformation can be referred to as $T(t)$. A reference motion state can then be generated (e.g., based on a slice with timestamp $t = t_0$) and so we can therefore take $T(t_0) = Id$ (wherein $Id$ = the reference motion state). The slice/image at $t = 0$ can be referred to as the ground truth. Each slice of the stack of slices (acquired during the CT scan) then corresponds to a certain time,

$$t_i = t_0 + i\Delta \tag{1}$$

It can then be assumed that each slice, i, is free of deformation, i.e., it corresponds to an (oblique) slice of the ground truth transformed rigidly by $T(t_i)$. In this example, three different CT scan volumes are obtained, A, B, and C, respectively. The slices of volume A are denoted by $a_i$, the slices of volume B by $b_i$, etc. Each volume is a representation of the ground truth transformed by $T(t)$, but the mapping of slices $i$ to times $t_{i,A}$, $t_{i,B}$, $t_{i,C}$ differs. Each volume has a different $t_0$ - these can be denoted as $t_{0,A}$, $t_{0,B}$, $t_{0,C}$.

Each of the volumes, A, B, and C can thus be transformed into the ground truth image by a slice-wise application of a (yet unknown) inverse of the rigid transformation $T$ taken at the time that corresponds to the respective time of the slice. To be clear, the rigid transformation function described in relation to method 100 of Fig. 1, can be considered an inverse of a rigid transformation function which transforms a volume of a ground truth representation into a motion-affected volume.

Thus, the desired reconstruction algorithm can be considered as an optimization (registration) problem, namely, to find a time-dependent rigid transformation $T^{-1}(t)$ such that the volumes spanned by $\{T^{-1}(t_{i,A})a_i\}$, $\{T^{-1}(t_{i,B})b_i\}$, and $\{T^{-1}(t_{i,C})c_i\}$ match. It should be noted that, as is usually done in registration problems, a smoothness constraint can be added to the rigid transformation $T^{-1}(t)$, i.e., such that the six parameters which describe the transformation vary smoothly in time.

This is thus a general description of an algorithm which works on entire scan volumes (e.g., simultaneously). Practically, however, an incremental version of the algorithm can be used, as will now be outlined.

As stated, individual times $t_{i,A}$, $t_{i,B}$, and $t_{i,C}$ only differ by a constant, e.g.:

$$t_{i,A} + 2\Delta = t_{i,B} + \Delta = t_{i,C} \tag{2}$$

For the sake of clarity, the time difference $\Delta$ is assumed to relate exactly to a slice index difference of $n$. The volume C can be assumed to be earliest in time, i.e., reconstructed from the detector segment that is on the leading edge of the detector. The time for the first reconstructed slice in volume C is set to $t_0$. Thus, subsequent slices of volume C correspond to times $t_1$, $t_2$, etc. Slices $b_0$ and $a_0$ thus correspond to times $t_n$ and $t_{2n}$, respectively.

An example method for compensating for motion during a CT scan can thus be outlined as follows:

$$\text{Set } T(t_0) = Id$$

Estimate $T^{-1}(t_n)$ by picking slice $b_0$, including (for registration purposes) their nearest neighbors, and estimating $T^{-1}(t_n)$ by rigid registration of the two slices from volume $B$ to $c_0$ (i.e., $b_0$'s neighbor slice, $b_1$)

Estimate $T^{-1}(t_{2n})$ by picking slices $b_n$ and $a_0$, including (for registration purposes) their nearest neighbors, and estimating, by rigid registration of the slices from volume $B$ to $c_n$ and the slices from volume $A$ to $b_0$, an intermediate transformation $S$ (setting $T^{-1}(t_{2n}) = S \circ T^{-1}(t_n)$), where $\circ$ denotes the concatenation of transformations.

For $n > 1$, the process leaves temporal gaps in the estimated transformations (e.g., in steps 2 and 3) - these intermediate transformations $T^{-1}(t_n), ..., T^{-1}(t_{n-1})$ may be obtained via interpolation between $T^{-1}(t_0)$ and $T^{-1}(t_n)$, etc.

Estimate $T^{-1}(t_{3n})$ by picking slices $b_{2n}$ and $a_n$, including (for registration purposes) their nearest neighbors $a_{n-1}$, $a_{n+1}$, and estimating, by rigid registration of the slices from volume $A$ to $b_n$, another intermediate transformation S (setting $T^{-1}(t_{3n}) = S \circ T^{-1}(t_{2n})$))

Iteratively repeat steps 3 to 5, *mutatis mutandis*, working through the volumes

Generate ground truth images (i.e., motion-compensated images) by applying the inverse transformation $T^{-1}$ to all slices and resampling to a cartesian grid. This step can be performed in a sliding fashion.

In the above example, images are generated in the ground truth motion state, i.e., the final reconstruction aims at representing the true geometry of the volume, e.g., a head. Alternatively, slice-wise motion state referencing can be used. For example, this can be simple to realize (in the simplified version of the algorithm described above, e.g., working on entire scan volumes) since it is equivalent to applying only local transformations S rather than accumulating the different rigid transformations (by concatenating S with previously estimated transformations $T^{-1}(t_{m,n})$. Additionally, the process can start with the identity transformation for the first slice of volume B.

In more detail, mathematically, the general concept can be considered an optimization problem for the unknown transformation $T^{-1}(t)$, sampled at some discrete time points $t_i$. For the sake of brevity, we assume $t_{i,A}=t_i$. Thus, we have $t_{i,B} = t_{i+\Delta}$ and $t_{i,C} = t_{i+2\Delta}$.

For each time point, a rigid transformation can be estimated, which can be specified by a six-dimensional parameter vector $\vec{p}_i$. For example, the elements of $\vec{p}_i$ can be three translations and three Euler angles.

For the sake of a compact notation, $T_{\vec{p}i}$ can be defined as the rigid transformation defined by $\vec{p}_i$. Using this nomenclature, the volumes to match during registration are spanned by $\{T_{\vec{p}_i}^{-1}a_i\}$, $\{T_{\vec{p}_{i+\Delta}}^{-1}b_i\}$, and $\{T_{\vec{p}_{i+2\Delta}}^{-1}c_i\}$. Of course, each of these volumes are now spanned by a couple of oblique slices (since different rigid transformations are applied to each slice of the stack. For the registration, it is thus convenient to also define versions of these volumes resampled to a common grid. These volumes can be defined with a resampling operator R such that the following volumes are three-dimensional images defined on the same voxel grid:

$$R\big(\{T_{\vec{p}_i}^{-1}a_i\}\big), R\big(\{T_{\vec{p}_{i+\Delta}}^{-1}b_i\}\big), R\big(\{T_{\vec{p}_{i+2\Delta}}^{-1}c_i\}\big) \tag{3}$$

The optimization performed during the registration can thus be written using a so-called data term:

$$D(\vec{p}) = \left\|R\big(\{T_{\vec{p}_i}^{-1}a_i\}\big) - R\big(\{T_{\vec{p}_{i+\Delta}}^{-1}b_i\}\big)\right\|^2 + \left\|R\big(\{T_{\vec{p}_i}^{-1}a_i\}\big) - R\big(\{T_{\vec{p}_{i+2\Delta}}^{-1}c_i\}\big)\right\|^2$$
$$+ \left\|R\big(\{T_{\vec{p}_i}^{-1}b_i\}\big) - R\big(\{T_{\vec{p}_{i+2\Delta}}^{-1}c_i\}\big)\right\|^2 \tag{4}$$

In equation (4), each of the terms is a sum of squared difference between two transformed and resampled volumes.

The smoothness constraint can be implemented, for instance, by using a so-called regularization term that ensures that the transformation parameters do not change quickly in time, for example, a regularization term of the form:

$$\Omega(\vec{p}) = \sum_i \|\vec{p}_i - \vec{p}_{i+1}\|_W^2 \tag{5}$$

may be used where the $W$-norm on the parameter space is defined by a diagonal matrix W with positive diagonal elements $w_1, w_2, w_3, w_4, w_5, w_6$ as

$$\|\vec{x}\|_w^2 = \sum_{k=1}^6 w_k x_k^2 \tag{6}$$

Thus, the overall registration process can be defined as a minimization problem according to:

$$\vec{P} = \operatorname{argmin}_{\vec{p}}\big(D(\vec{p}) + \lambda\Omega(\vec{p})\big) \tag{7}$$

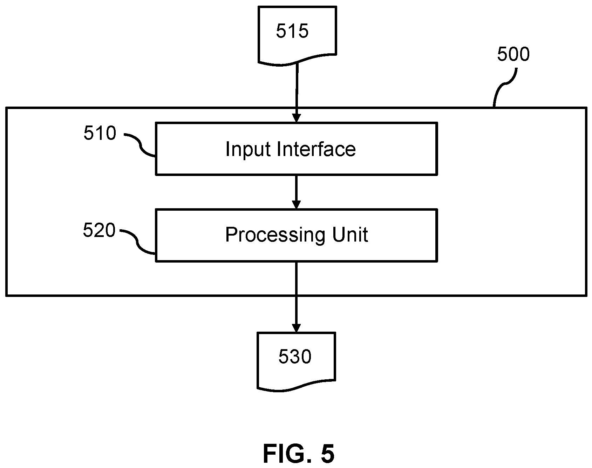

Referring now to Fig. 5, there is depicted a system 500 for compensating for motion during a CT scan according to a proposed embodiment. The system 500 comprises an input interface 510 and a processing unit 520.

The system 500 is configured to compensate for motion during a CT scan by processing inputs 515. The inputs 515 comprise a first CT scan volume of a portion of a subject comprising a first stack of slices separated in time, and a second CT scan volume of the same portion of a subject comprising a second stack of slices separated in time, wherein the second CT scan volume partially overlaps in time with the first CT scan volume. The input interface 510 receives the inputs 515 and the processing unit 520 is then configured to process them to generate an output 530. The output 530 comprises a motion-compensated CT scan volume. The processing unit 520 is specifically configured to do this by: generating a reference motion state based on one of the slices from the first stack of slices; determining a time-dependent rigid transformation function based on the reference motion state and the first and second CT scan volumes; and generating a motion-compensated CT scan volume based on the rigid transformation function and either the first and second CT scan volumes or projection data from which the first and second CT scan volumes were reconstructed.

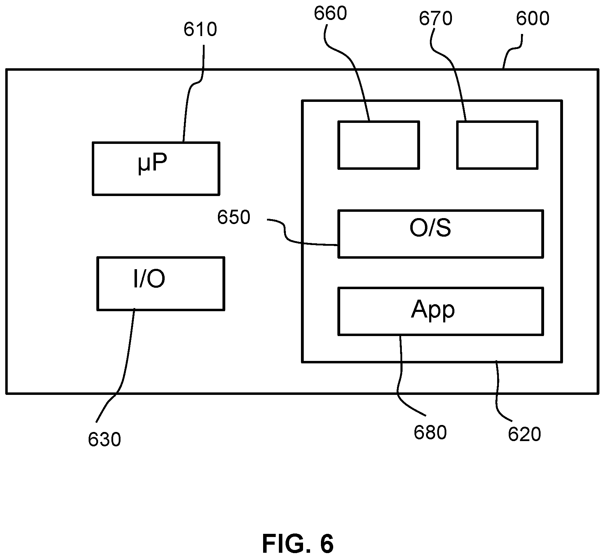

Fig. 6 illustrates an example of a computer 600 within which one or more parts of an embodiment may be employed. Various operations discussed above may utilize the capabilities of the computer 600. In this regard, it is to be understood that system functional blocks can run on a single computer or may be distributed over several computers and locations (e.g. connected via internet).

The computer 600 includes, but is not limited to, PCs, workstations, laptops, PDAs, palm devices, servers, storages, and the like. Generally, in terms of hardware architecture, the computer 600 may include one or more processors 610, memory 620 and one or more I/O devices 630 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 610 is a hardware device for executing software that can be stored in the memory 620. The processor 610 can be virtually any custom made or commercially available processor, a central processing unit (CPU), a digital signal processor (DSP), or an auxiliary processor among several processors associated with the computer 600, and the processor 610 may be a semiconductor based microprocessor (in the form of a microchip) or a microprocessor.

The memory 620 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 620 may incorporate electronic, magnetic, optical, and/or other types of storage media. Note that the memory 620 can have a distributed architecture, where various components are situated remote from one another, but can be accessed by the processor 610.

The software in the memory 620 may include one or more separate programs, each of which comprises an ordered listing of executable instructions for implementing logical functions. The software in the memory 620 includes a suitable operating system (O/S) 650, compiler 660, source code 670, and one or more applications 680 in accordance with exemplary embodiments. As illustrated, the application 680 comprises numerous functional components for implementing the features and operations of the exemplary embodiments. The application 680 of the computer 600 may represent various applications, computational units, logic, functional units, processes, operations, virtual entities, and/or modules in accordance with exemplary embodiments, but the application 680 is not meant to be a limitation.

The operating system 650 controls the execution of other computer programs, and provides scheduling, input-output control, file and data management, memory management, and communication control and related services. It is contemplated by the inventors that the application 680 for implementing exemplary embodiments may be applicable on all commercially available operating systems.

Application 680 may be a source program, executable program (object code), script, or any other entity comprising a set of instructions to be performed.

The I/O devices 630 may include input devices such as, for example but not limited to, a mouse, keyboard, scanner, microphone, camera, etc. Furthermore, the I/O devices 630 may also include output devices, for example but not limited to a printer, display, etc. Finally, the I/O devices 630 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 630 also include components for communicating over various networks, such as the Internet or intranet.

When the computer 600 is in operation, the processor 610 is configured to execute software stored within the memory 620, to communicate data to and from the memory 620, and to generally control operations of the computer 600 pursuant to the software. The application 680 and the O/S 650 are read, in whole or in part, by the processor 610, perhaps buffered within the processor 610, and then executed.

When the application 680 is implemented in software it should be noted that the application 680 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The methods of Figs. 1, 3 and 4, and the system of Fig. 5, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Fig. 6 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions, the architecture, functionality, and operation of possible implementations of systems, methods, and computer program products according to various embodiments of the present invention.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Implementations in accordance with the present disclosure relate to various techniques, methods, schemes and/or solutions pertaining to compensating for motion during a CT scan. According to proposed concepts, a number of possible solutions may be implemented separately or jointly. That is, although these possible solutions may be described below separately, two or more of these possible solutions may be implemented in one combination or another.

**Claims**

1. A computer-implemented method (100) for compensating for motion during a CT scan, the method comprising:

   receiving a first CT scan volume (110) of a portion of a subject comprising a first stack of slices separated in time;
   receiving a second CT scan volume (120) of the same portion of a subject comprising a second stack of slices separated in time, wherein the second CT scan volume partially overlaps in time with the first CT scan volume;
   generating a reference motion state (130) based on one of the slices from the first stack of slices;
   determining a time-dependent rigid transformation function (140) based on the reference motion state and the first and second CT scan volumes; and

generating a motion-compensated CT scan volume (150) based on the rigid transformation function and either the first and second CT scan volumes or projection data from which the first and second CT scan volumes were reconstructed.

2. The computer-implemented method of claim 1, wherein determining the rigid transformation function comprises generating a first estimate of the rigid transformation function (340) based on the reference motion state and a first neighboring slice in the second stack of slices neighboring the slice upon which the reference motion state was based.

3. The computer-implemented method of claim 2, wherein determining the rigid transformation function further comprises generating a second estimate (342) based on the first estimate, a first corresponding slice in the first stack of slices corresponding in time to the first neighboring slice, and a second neighboring slice in the second stack of slices neighboring the first corresponding slice.

4. The computer-implemented method of claim 3, wherein determining the rigid transformation function (344) further comprises interpolating between the first estimate and the second estimate.

5. The computer-implemented method of any of claims 1 to 4, wherein generating a motion-compensated CT scan volume comprises:

   compensating for motion in each slice of the first and second stacks of slices respectively (445) based on the rigid transformation function to generate motion-compensated first and second CT scan volumes; and
   generating a motion-compensated CT scan volume (450) based on the motion-compensated first and second CT scan volumes.

6. The computer-implemented method of any of claims 1 to 4, wherein generating a motion-compensated CT scan volume comprises:

   receiving projection data (315) from which the first and second CT scan volumes were reconstructed; and
   reconstructing a motion-compensated CT scan volume (350) based on the projection data and the rigid transformation function.

7. The computer-implemented method of any of claims 1 to 6, wherein the first stack of slices comprises at least one slice which is earlier than any slice in the second stack of slices, and the reference motion state is generated based on one of these earlier slices.

8. The computer-implemented method of any of claims 1 to 7, wherein determining the rigid transformation function comprises estimating the second CT scan volume to be separated in time from the first CT scan volume by a constant corresponding to an integer number of slices.

9. The computer-implemented method of any of claims 1 to 8, wherein generating the reference motion state comprises selecting one of the slices from the first stack of slices as the reference motion state (330), and optionally, transforming the selected slice.

10. The computer-implemented method of any of claims 1 to 9, wherein a rigid transformation comprises a translation and/or a rotation without any scaling or deformation.

11. The computer-implemented method of any of claims 1 to 10, wherein the portion of the subject comprises a rigid portion of the subject.

12. The method of claim 11, wherein the rigid portion of subject is the head of the subject.

13. A computer program product comprising computer program code means which, when executed on a computing device having a processing system, cause the processing system to perform all of the steps of the computer-implemented method according to any of claims 1 to 12.

14. A system (500) for compensating for motion during a CT scan, the system comprising:
   an input interface (510) configured to:

receive a first CT scan volume of a portion of a subject comprising a first stack of slices separated in time; and
receive a second CT scan volume of the same portion of a subject comprising a second stack of slices separated in time, wherein the second CT scan volume partially overlaps in time with the first CT scan volume; and
a processing unit (520) configured to:

generate a reference motion state based on one of the slices from the first stack of slices;
determine a time-dependent rigid transformation function based on the reference motion state and the first and second CT scan volumes; and
generate a motion-compensated CT scan volume based on the rigid transformation function and either the first and second CT scan volumes or projection data from which the first and second CT scan volumes were reconstructed.

15. The system of claim 14, wherein determining the rigid transformation function comprises generating a first estimate of the rigid transformation function based on the reference motion state and a first neighboring slice in the second stack of slices neighboring the slice upon which the reference motion state was based.

100

110

Receiving first CT scan
volume

120

Receiving second CT scan
volume

130

Generating reference
motion state

140

Determining rigid
transformation function

150

Generating motion-
compensated CT scan
volume

FIG. 1

210a  210b  210c

A  B  C

$$T^{-1}(t_{i,A})a_i \qquad T^{-1}(t_{i,B})b_i \qquad T^{-1}(t_{i,C})c_i$$

220a  220b  220c

**FIG. 2A**

260    250

$T(t_{3n+1})$     $T(t_{2n+1})$

$T(t_{3n})$     $T(t_{2n})$

$T(t_{3n-1})$     $T(t_{2n-1})$

...     ...

$T(t_{2n+1})$     $T(t_{n+1})$

$T(t_{2n})$     $T(t_n)$

$T(t_{2n-1})$     $T(t_{n-1})$

...     ...

$T(t_{n+1})$     $T(t_1)$

265    $T(t_n)$     $\xrightarrow{1}$     $T(t_0) = \mathrm{Id}$     255

260    250

$T(t_{3n+1})$     $T(t_{2n+1})$

$T(t_{3n})$     $T(t_{2n})$

$T(t_{3n-1})$     $T(t_{2n-1})$

...     ...

$T(t_{2n+1})$     $T(t_{n+1})$

285    $T(t_{2n})$     $\xrightarrow{2}$     $T(t_n)$     275

$T(t_{2n-1})$     $T(t_{n-1})$

...     ...

$T(t_{n+1})$     $T(t_1)$

$T(t_n)$     $T(t_0) = \mathrm{Id}$

# FIG. 2B

**FIG. 3**

400

110
Receiving first CT scan volume

120
Receiving second CT scan volume

130 — Generating reference motion state

140 — Determining rigid transformation function

445 — Compensating for motion in each slice of first and second CT scan volumes

450 — Generating motion-compensated CT scan volume

**FIG. 4**

**FIG. 5**

**FIG. 6**

EUROPEAN SEARCH REPORT

Application Number

EP 24 21 7642

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2006/165267 A1 (WYMAN BRADLEY [US] ET AL) 27 July 2006 (2006-07-27) | 1-3,5-15 | INV.<br>G06T11/00 |
| A | * paragraphs [0049] - [0067]; figures 1-4 * | 4 | G06T7/20<br>A61B6/03 |
| | ----- | | |
| T | STUDHOLME C ET AL: "AUTOMATED 3-D REGISTRATION OF MR AND CT IMAGES OF THE BAND", MEDICAL IMAGE ANALYSIS, OXFORDUNIVERSITY PRESS, OXFORD, GB, vol. 1, no. 2, 1 June 1996 (1996-06-01), pages 163-175, XP002942556, ISSN: 1361-8423, DOI: 10.1016/S1361-8415(96)80011-9 * the whole document * | 2,3,15 | |
| | ----- | | |
| A | US 10 249 064 B2 (TOSHIBA MEDICAL SYS CORP [JP]) 2 April 2019 (2019-04-02) * the whole document * | 1-15 | |
| | ----- | | |
| A | US 2017/340304 A1 (QIULIN TANG [US] ET AL) 30 November 2017 (2017-11-30) * the whole document * | 1-15 | TECHNICAL FIELDS SEARCHED (IPC)<br><br>G06T<br>A61B |
| | ----- | | |

The present search report has been drawn up for all claims

| Place of search | Date of completion of the search | Examiner |
|---|---|---|
| Munich | 6 May 2025 | Tillier, Christophe |

CATEGORY OF CITED DOCUMENTS

X : particularly relevant if taken alone
Y : particularly relevant if combined with another
　　document of the same category
A : technological background
O : non-written disclosure
P : intermediate document

T : theory or principle underlying the invention
E : earlier patent document, but published on, or
　　after the filing date
D : document cited in the application
L : document cited for other reasons
......................................................................................................
& : member of the same patent family, corresponding
　　document

EPO FORM 1503 03.82 (P04C01)

## ANNEX TO THE EUROPEAN SEARCH REPORT
## ON EUROPEAN PATENT APPLICATION NO.

EP 24 21 7642

This annex lists the patent family members relating to the patent documents cited in the above-mentioned European search report.
The members are as contained in the European Patent Office EDP file on
The European Patent Office is in no way liable for these particulars which are merely given for the purpose of information.

06-05-2025

| Patent document cited in search report | | Publication date | Patent family member(s) | | Publication date |
|---|---|---|---|---|---|
| US 2006165267 | A1 | 27-07-2006 | NONE | | |
| US 10249064 | B2 | 02-04-2019 | JP | 6985056 B2 | 22-12-2021 |
| | | | JP | 2018020120 A | 08-02-2018 |
| | | | US | 2018040145 A1 | 08-02-2018 |
| US 2017340304 | A1 | 30-11-2017 | JP | 6925868 B2 | 25-08-2021 |
| | | | JP | 2017209498 A | 30-11-2017 |
| | | | US | 2017340304 A1 | 30-11-2017 |

EPO FORM P0459

For more details about this annex : see Official Journal of the European Patent Office, No. 12/82